# EUROPEAN PATENT APPLICATION

(11) **EP 2 243 468 A1**
(43) Date of publication of application: **27.10.2010**
(21) Application number: 10160229.0
(22) Date of filing: 16.04.2010
(51) Int. Cl.: A61K 9/00, A61K 31/444

(54) **Orally Disintegrating Dimebolin Compositions**

(30) Priority: 17.04.2009 TR 200903014
(71) Applicant: Sanovel Ilac Sanayi ve Ticaret A.S., 34398 Istanbul (TR)
(72) Inventor: Toksöz, Ahmet, 34398, Istanbul (TR); Cifter, Ümit, 34398, Istanbul (TR); Türkyilmaz, Ali, 34398, Istanbul (TR)
(74) Representative: Sevinç, Erkan

(57) **Abstract**

According to the present invention an orally disintegrating composition comprising dimebolin or a pharmaceutically acceptable salt thereof together with one or more pharmaceutically acceptable excipients is provided.

## Description

### Technical Aspect

The present invention is related to an orally disintegrating composition comprising dimebolin or a pharmaceutically acceptable salt thereof together with one or more pharmaceutically acceptable excipients.

### Background of the Invention

Dimebolin is an antiallergic agent and N-methyl d-aspartat receptor antagonist (NMDA) receptor antagonist. Its chemical name is 2,8-dimethyl-5-(2-(6-methyl-3-pyridyl)ethyl)-2,3,4,5-tetrahydro-1H-pyrido[4,3-b]indole and its chemical structure is shown in the Formula I.

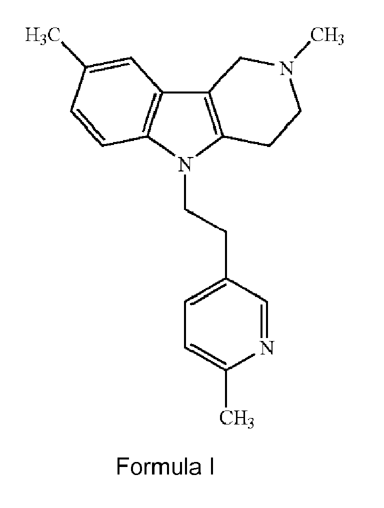

Dimebolin has been used as an antiallergic agent in Russia for over 20 years. It has hereto been administered orally in the form of tablets containing a dose from 2.5 mg to 20 mg. The usual prescription is 1 tablet three times a day.

As described in U.S. Patent No. 6,187,785 and 7,071,206, hydrogenated pyrido[4,3-b]indole derivatives, such as dimebolin, have NMDA receptor antagonist properties, which make them useful for treating neurodegenerative diseases, such as Alzheimer's disease. As described in WO 2005/055951, hydrogenated pyrido[4,3-b]indole derivatives, such as dimebolin, are useful as human or veterinary geroprotectors. They work by delaying the onset and/or development of an age-associated or related manifestation and/or pathology or condition, including disturbance in skin-hair integument, vision disturbance and weight loss. U.S. Provisional Patent Application No. 60/723,403, filed October 4, 2006, and U.S. Patent Application No. 11/543,529, filed October 4, 2006, disclose hydrogenated pyrido[4,3- b] indole derivatives, such as dimebolin, as neuroprotectors for use in treating and/or preventing and/or slowing the progression or onset and/or development of Huntington's disease. RU application which is filed January 25, 2006 with title of "Agent for Treatment of Schizophrenia Based on Hydrogenated Pyrido [4,3 -b] indoles (Variations), a Pharmacological Agent Based on it, and a Method of Using it" is another publication in this field.

It is desirable in the treatment of a number of diseases, to provide the active pharmaceutical ingredient in an orally disintegrating form. They are advantageous for administration of medicaments to patients such as young, elder, and non-compliant patient. Orally disintegrating dosage forms are convenient for situations where potable water may not be readily available or desirable. Medicaments which may be used for sedatives, hypnotics, and antipsychotic are amendable such dosage forms.

It is difficult to develop orally disintegrating compositions because of several different reasons. First of all, the time in which dosage form must disintegrate in the oral cavity with the existence of saliva has to be much shorter than it should be in stomach. So those compositions are very porous and thus not very hard. These porous compositions tend to be very sensitive to humidity. As a consequence, they may have some stability problems. Additionally, orally disintegrating compositions need to take precautions in the preparation, packaging, handling and storing of the finished dosage forms.

Accordingly, a need rises for orally disintegrating compositions of dimebolin or pharmaceutically acceptable salt, which overcomes above described problems and which further provide the advantageous property of allowing the active medicament to be disintegrate or dissolve in the oral cavity.

### Description of the invention

According to the present invention an orally disintegrating composition comprising dimebolin or a pharmaceutically acceptable salt thereof together with one or more pharmaceutically acceptable excipients is provided.

In one embodiment the amount of dimebolin or a pharmaceutically acceptable salt thereof is present in about 1 to 80% by weight of total composition, preferably about 25 to 80%, the more preferably about 25 to 35%. Dimebolin is preferably in the form of an HCl salt.

In a further aspect, the present invention relates to the orally disintegrating compositions comprising dimebolin or a pharmaceutically acceptable salt thereof, is in an amount of 1 mg to 100mg.

The compositions of the invention comprise one or more excipients. Such excipients include super disintegrants, sweeteners, diluents, binders, preservatives, artificial agents, glidants and lubricants.

Suitable super disintegrants may include but not limited to starch, sodium starch glycollate, low-substituted hydroxypropylcellulose and the like and mixtures thereof, preferably low-substituted hydroxypropylcellulose.

In preferred embodiments, the amount of low-substituted hydroxypropylcellulose is present in about 0.5 to 30% by weight of total composition, particularly about 3 to 10%.

Suitable sweeteners may include but not limited to aspartame, sucralose, saccharin, glucose, fructose, sugar alcohols e.g. mannitol, sorbitol, xylitol, erythritol and the like and mixtures thereof, preferably mannitol and aspartam.

In other preferred embodiments of present invention, the amount of mannitol is present in about 1 to 60% by weight of total composition, preferably about 10 to 40%.

It has unexpectedly found that this orally disintegrating composition having a weight ratio of low-substituted hydroxypropylcellulose to mannitol between the range of 1:100 to 10:1 (w/w) has positive effect over the disintegration time.

Suitable diluents may include but not limited to lactose, microcrystalline cellulose, starch, sodium carbonate, sodium bicarbonate and the like and mixtures thereof; preferably microcrystalline cellulose.

Suitable binders may include but not limited to povidone, polyethylene glycol, cellulose derivatives such as hydroxypropyl methyl cellulose, carboxy methyl cellulose, methyl cellulose, crospovidone, gelatin, polyvinylalcohol, carrageenan, guar gum, xanthan gum croscarmellose sodium and the like and mixtures thereof; preferably croscarmellose sodium.

Suitable preservatives such as antimicrobiyal and antioxidants may include but not limited to methyl paraben, propyl paraben, butylated hydroxytoluene, butylated hydroxyanisole and the like and mixtures thereof.

Suitable artificial agents may include but not limited to mint (e.g. mint sugar, etc.), menthol, cinnamon, vanilla, artificial vanilla, chocolate, artificial chocolate, natural and artificial fruit essences (e.g. cherry, grape, orange, strawberry, etc.) and the like and mixtures thereof, preferably vanilla.

Suitable glidants may include but not limited to colloidal silicon dioxide, talc, aluminium silicate and the like and mixtures thereof, preferably colloidal silicon dioxide.

Suitable lubricants may include but not limited to magnesium strearate, calcium stearate, sodium stearyl fumarate, sodium lauryl sulphate, stearic acid and the like and mixtures thereof, preferably magnesium strearate .

The orally disintegrating composition is such that it disintegrates within up to 90 seconds, preferably 40 seconds, the more preferably 25 seconds. The disintegration process takes place in oral cavity. In order to meet above mentioned advantageous of the composition, the amount of low-substituted hydroxypropylcellulose is present in about 0.5 to 30% by weight of total composition, preferably about 3 to 10% or the amount of mannitol is present in about 1 to 60% by weight of total composition, preferably about 10 to 40%.

The composition comprising (a) about 3 to 10 % by weight of low-substituted hydroxypropylcellulose, (b) about 10 to 40% by weight of mannitol, (c) about 4 to 60 % by weight of microcrystalline cellulose, (d) about 0.5 to 15% by weight of croscarmellose sodium, (e) about 0.1 to 5% by weight of aspartame, (f) about 0.1 to 6% by weight of vanilla, (g) about 0.1 to 5% by weight of colloidal silicon dioxide, (h) about 0.1 to 5% by weight of magnesium stearate in order to be orally disintegrating and be hard enough for transporting and commercializing.

The present invention provides an orally disintegrating composition of dimebolin or a pharmaceutically acceptable salt thereof together with one or more pharmaceutically acceptable excipients which is stable throughout the shelflife and which has high bioavailability.

The orally disintegrating composition of the invention includes tablet, sachet, minitablet, pellet, which can be formulated in accordance with methods that are standard in the art.

The orally disintegrating compositions of the present invention may be prepared by conventional technology well known to those skilled in the art such as wet granulation, direct compression, dry granulation and the like. The orally disintegrating compositions of the present invention may also be prepared by other technologies such as zydis, orasolv, durasolv, wowtab and the like. Preferably dimebolin, or a pharmaceutically acceptable salt thereof, and other excipients are mixed together then the mixture is compressed to form tablets.

The manufacturing process is preferably performed by mixing the components, blending the mixture with glidant(s) and lubricant(s) and compressing the blended mixture to form tablets.

A preferred process for preparing the orally disintegrating compositions of the invention comprises the following steps:
(a) mixing dimebolin, or a pharmaceutically acceptable salt thereof other excipients;
(b) blending the mixture with a glidant and lubricant;
(c) compressing the blended mixture to form tablets.

The invention is further defined by reference to the following example. Although the example is not intended to limit the scope of the present invention, it should be considered in the light of the description detailed above.

### Example

### Dimebolin orally disintegrating tablet

This example describes the orally disintegrating tablet comprising dimebolin or a pharmaceutically acceptable salt thereof. Active ingredient and excipients of the tablet are given below:

### Dimebolin 20 mg orally disintegrating tablet

| **Active ingredient** | |
|---|---|
| Dimebolin | **20.00 mg** |
| **Excipients** | |
| Low-substituted hydroxypropylcellulose | 4.00 mg |
| LH-11 | |
| Spray dried mannitol | 22.80 mg |
| Microcrystalline cellulose PH 112 | 55.00 mg |
| Croscarmellose sodium | 9.00 mg |
| Aspartam | 0.20 mg |
| Vanilla | 3.00 mg |
| Colloidal Silicon Dioxide | 0.50 mg |
| Magnesium Stearate | 1.5 mg |
| **Total** | **120 mg** |

The disintegration time of the five Dimebolin 20 mg orally disintegrating tablet comprising above excipients are measured by the standard disintegration method in US pharmacopeia (USP disintegration test <701>). The results are given in Table 1.

**Table 1. Disintegration time of Dimebolin 20 mg orally disintegrating tablet**

| | 1. tablet | 2. tablet | 3. tablet | 4. tablet | 5. tablet |
|---|---|---|---|---|---|
| Time (sec.) | 20 | 22 | 24 | 21 | 22 |

Therefore, further aspects of the present invention concern the use of pharmaceutical compositions comprising dimebolin or a pharmaceutically acceptable salt thereof, together with one or more pharmaceutically acceptable excipients for the manufacture of a medicament for the treatment of Alzheimer disease, neuropathic pain, chronic visceral pain, chronic inflammatory pain, headache, antihistaminic disease in a warm- blooded animal.

## Claims

1. An orally disintegrating composition comprising dimebolin or a pharmaceutically acceptable salt thereof together with one or more pharmaceutically acceptable excipients.

2. The orally disintegrating composition according to claim 1 wherein the composition disintegrates within up to 90 seconds, preferably 40 seconds in oral cavity.

3. The orally disintegrating composition according to claim 1-2 wherein dimebolin or a pharmaceutically acceptable salt thereof is present in about 1 to 80%, preferably about 25 to 80% by weight of total composition.

4. The orally disintegrating composition according to claim 1-3 wherein the amount of dimebolin or a pharmaceutically acceptable salt is 1 mg to 100mg.

5. The orally disintegrating composition according to claims 1-4 wherein dimebolin is in the form of an HCl salt.

6. The orally disintegrating composition according to claims 1-5 wherein the one or more pharmaceutically acceptable excipients are selected from the group comprising super disintegrants, sweeteners, diluents, binders, preservatives, artificial agents, lubricants, and glidants.

7. The orally disintegrating composition according to claim 1 and 6, wherein the disintegrant is low-substituted hydroxypropylcellulose.

8. The orally disintegrating composition according to claim 1, 6 and 7 wherein the amount of low-substituted hydroxypropylcellulose is present in about 0.5 to 30% by weight of total composition.

9. The orally disintegrating composition according to claim 1, 7 and 8 wherein the amount of low-substituted hydroxypropylcellulose is about 3 to 10% by weight of total composition.

10. The orally disintegrating composition according to claim 1 and 6, wherein the sweeteners are mannitol and aspartam.

11. The orally disintegrating composition according to claim 1, 6 and 10 wherein the amount of mannitol is about 1 to 60% by weight of total composition.

12. The orally disintegrating composition according to claim 1, 10 and 11 wherein the amount of mannitol is about 10 to 40% by weight of total composition.

13. The orally disintegrating composition according to any preceding claim, wherein the weight ratio of low-substituted hydroxypropylcellulose to mannitol is in the range of between 1:100 to 10:1 (w/w).

14. The orally disintegrating composition according to any preceding claim comprising (a) about 3 to 10 % by weight of low-substituted hydroxypropylcellulose, (b) about 10 to 40% by weight of mannitol, (c) about 4 to 60 % by weight of microcrystalline cellulose, (d) about 0.5 to 15% by weight of croscarmellose sodium, (e) about 0.1 to 5% by weight of aspartame, (f) about 0.1 to 6% by weight of vanilla, (g) about 0.1 to 5% by weight of colloidal silicon dioxide, (h) about 0.1 to 5% by weight of magnesium stearate.

15. A process for preparing orally disintegrating compositions according to any preceding claim which comprises
(a) mixing dimebolin, or a pharmaceutically acceptable salt thereof other excipients;
(b) blending the mixture with a glidant and lubricant;
(c) compressing the blended mixture to form tablets.
